# EUROPÄISCHE PATENTANMELDUNG

(11) **EP 2 965 710 A1**
(43) Veröffentlichungstag der Anmeldung: **13.01.2016**
(21) Anmeldenummer: 14176188.2
(22) Anmeldetag: 08.07.2014
(51) Int. Cl.: A61C 5/00, A61C 9/00, A61C 13/00, A61C 13/08, A61C 13/20

(54) **Individualisierte Negativformen**

(71) Anmelder: Coltène/Whaledent AG, 9450 Altstaetten (CH)
(72) Erfinder: Sutter, Simon, 7000 Chur (CH); Schaufelberger, Martin, 8872 Weesen (CH)
(74) Vertreter: Hepp Wenger Ryffel AG

(57) **Zusammenfassung**

Die Erfindung betrifft ein Verfahren zur Herstellung einer individuellen, insbesondere mehrteiligen, Negativform für die Herstellung von Zahnersatz, eine derartige Negativform, ein Verfahren zur Herstellung von individualisiertem Zahnersatz sowie einen derartigen Zahnersatz.

## Beschreibung

Die Erfindung betrifft ein Verfahren zur Herstellung einer individualisierten, insbesondere mehrteiligen, Negativform, eine derartige Negativform, ein Verfahren zur Herstellung eines Zahnersatzes mittels einer individualisierten, insbesondere mehrteiligen, Negativform sowie einen derartigen Zahnersatz.

Fehlende Zahnhartsubstanz wird heute in der Regel entweder durch den Zahnarzt direkt mit Komposit-Materialien oder indirekt mit im Labor vorgefertigten Kronen, Inlays oder Veneers restauriert.

Bei der direkten Restauration werden Komposit-Pasten schichtweise aufgebaut, jede Schicht für sich mit Lichtbestrahlung *in situ* ausgehärtet und anschliessend die komplette Restauration poliert. Bei der indirekten Restauration werden in einer ersten Sitzung eines Patienten bei einem Zahnarzt negative Abformungen erstellt, dabei sowohl Abformungen der ursprünglichen Zahnsituation und/oder der Situation nach einer Entfernung schadhafter Zahnsubstanz. Aus diesen beiden negativen Abformungen fertigt ein Zahntechniker einen angepassten Zahnersatz, welcher in einer zweiten Sitzung durch den Zahnarzt mit einem Zement am präparierten Zahn befestigt wird.

Seit einigen Jahren werden Abformungen nicht mehr ausschliesslich physisch durch Auftragen von Kunststoffmassen auf einen Zahn, Aushärten und Abnehmen der Abformung erhalten, sondern vermehrt auch durch digitales Scannen der Zahnsituation. Die resultierenden 3-dimensionalen Daten der geometrischen Zahnsituation können mit entsprechenden Softwarelösungen zu einem 3-dimensionalne Modell eines Zahnersatzes verarbeitet werden. Das 3-dimensionale Modell wird zur Herstellung eines Zahnersatzes an eine CNC-Fräsmaschine übermittelt und entsprechend aus einem Block eines Zahnersatzmaterials gefräst. Dabei sind jedoch meist mindestens zwei Sitzungen des Patienten beim behandelnden Zahnarzt erforderlich und dadurch wird die Behandlung zeit- und kostenintensiv.

An den bekannten Fräsverfahren ist nachteilig, dass sich aus vorgefertigten Zahnersatzblöcken vor allem nur einfarbige Restaurationen herstellen lassen, was einem natürlichen und ästhetischen Erscheinungsbild der Restauration nicht genügt. Dazu sollte die Restauration aus mindestens zwei Farben aufgebaut sein, beispielsweise einem opaken Kern und einem transluzentem Mantel. Zweifarbige Blöcke sind bekannt. Die Farbübergange lassen sich bei diesen jedoch nicht ausreichend definieren.

Alternative Verfahren zur Herstellung eines Zahnersatzes sind bekannt und umfassen auch aufbauende Verfahren wie beispielsweise 3D-Drucken, Lasersintern oder stereolithographische Verfahren. Derartige Verfahren sind in EP 1 240 878 Bl, EP 1 252 867 B1 und US 7,029,279 B2 offenbart. Nachteilig an diesen Verfahren sind jedoch, dass derzeit keine Materialien zur Verfügung, die den Anforderungen der DIN EN ISO 4049 für Zahnersatz genügen. Zudem weist der mittels aufbauenden Verfahren hergestellte Zahnersatz eine Material- und Herstellungs-bedingte Rauigkeit aufweist, die einen direkten Einsatz in eine Defektstelle unmöglich macht. Vielmehr sind weitere Polier- und Bearbeitungsschritte notwendig.

Bei indirekten Zahnrestaurationen besteht des Weiteren die Möglichkeit standardisierte Formen zu verwenden oder aber für den Patienten individuell angepasste Formen anzufertigen. Lösungen mit standardisierten Formen, wie beispielsweise Componeer® (Direct Composite Veneers, Coltene), bietet neben der raschen Patientenversorgung Kostenvorteile, da sie sich in einer einzelnen Sitzung einbringen lassen.

Standardisierte Negative sind ebenfalls bekannt, wie beispielsweise erhältlich als Frasaco-Stripkronen, welche ebenfalls eine kostengünstige und rasche Patientenversorgung erlauben.

Prinzipielle Nachteile an standardisierten Formen und Negativen sind die *per se* nicht-individuelle Form und Gestalt des in die Defektstelle eingebrachten Zahnersatzes.

EP 1 954 211 B1 beschreibt ein Verfahren zur Erzeugung einer dentalen Prothese mit einem Gerüst, einer Verblendung und einem Modell. Diese können mittels additiven Verfahren hergestellt sein und dienen zur Ausbildung einer Negativform, welche mit Zahnersatzmaterial befüllt werden kann. Die in die Defektsituation einzubringende Dentalprothese ist dementsprechend mehrteilig aufgebaut, bestehende aus einem Metall- oder Keramikgerüst, welches vorab angefertigt werden muss. In der Form wir dann die Verblendung des Gerüstes hergestellt. Dadurch ist das Verfahren aufwendig durchzuführen.

Es ist daher Aufgabe der Erfindung, die Nachteile des Stands der Technik zu überwinden. Insbesondere ist es eine Aufgabe der Erfindung Verfahren zur zeit- und kosteneffizienten Herstellung einer individualisierten, insbesondere mehrteiligen, Negativform sowie eines individualisierten Zahnersatzes bereitzustellen. Weitere Aufgaben der Erfindung sind es eine individualisierten, insbesondere mehrteiligen, Negativform sowie einen individualisierten Zahnersatz bereitzustellen.

Diese Aufgaben werden durch die in den unabhängigen Ansprüchen definierten Merkmale gelöst.

Die Erfindung betrifft ein Verfahren zur Herstellung einer individuellen, insbesondere mehrteiligen, Negativform für die Herstellung von Zahnersatz. Das Verfahren umfasst die Schritte
i) Erfassen digitaler Scandaten eines Zahns oder einer, insbesondere präparierten, Defektstelle insbesondere mittels eines Interoralscanners, und
ii) Herstellen der individualisierten, insbesondere mehrteiligen, Negativform mit einem computer-gesteuertem Verfahren, insbesondere einem CNC-Verfahren, basierend auf den erfassten Scandaten.

Zwischen dem vorgenannten Schritt i) und dem vorgenannten Schritt ii) wird bevorzugt der Schritt Konstruieren eines 3-dimensionalen Modells eines individualisierten Zahnersatzes und/oder eines 3-dimensionalen Modells der individualisierten, insbesondere mehrteiligen, Negativform, insbesondere mittels eines softwarebasierten Expertensystems, auf Basis der erfassen Scandaten durchgeführt werden.

Auf diese Weise wird ein kosten- und zeiteffizientes Verfahren zur Herstellung einer individualisierten, insbesondere mehrteiligen, Negativform für die Herstellung von Zahnersatz bereitgestellt. Eine individualisierte Negativform lässt sich mit dem Verfahren in kurzer Zeit herstellen, und mittels der erhaltenen Negativform lässt sich ein individualisierter Zahnersatz ebenfalls in kurzer Zeit herstellen. Somit erfordert das erfindungsgemässe Verfahren keine zweite Patientensitzung bei dem behandelnden Zahnarzt, da alle Verfahrensschritte bis hin zur Herstellung eines Zahnersatzes während einer einzigen Patientensitzung durchgeführt werden können. Idealerweise wären Dentalteile mittels Rapid Prototyping oder ähnlichen Verfahren direkt herstellbar. Dies ist allerdings nicht möglich, da sich mit 3D-Druckverfahren die derzeitigen Komposit-Materialien nicht verarbeiten lassen. Andererseits erfüllen Dentalteile hergestellt aus derzeit verfügbaren druckfähigen Materialien die notwendigen mechanischen Anforderungen nicht.

Zur digitalen Bearbeitung der im Verfahren erhaltenen Scandaten können diese mit geeigneten Softwarelösungen (Software-basierte Expertensysteme) insbesondere automatisiert bearbeitet werden. Dadurch werden die Parameter der herzustellenden Negativform optimiert und eine exakt an die Zahnsituation angepasste Zahnrestauration möglich.

Das computer-gesteuerte Verfahren, insbesondere das CNC-Verfahren, kann ein abtragendes Verfahren, insbesondere ein Fräs-, Schleif- oder Strahl-Verfahren, sein.

Unter CNC-Verfahren verstehen sich vorgängig, hier und im Folgenden Verfahren, welche auf Computerized Numerical Control basieren.

Das computer-gesteuerte Verfahren, insbesondere das CNC-Verfahren, kann ein additives Verfahren, insbesondere Stereolitographie, selektives Lasersintern, selektives Laserschmelzen, digital light processing, polyjet printing, multijet modeling, fused deposition molding, sein. Auf diese Weise wird die individualisierte, insbesondere mehrteilige Negativform zeiteffizient und mittels geringem Materialeinsatz hergestellt. Dies kann bei einigen der vorgenannten Verfahren Vorort während der einzigen Behandlungssitzung beim Zahnarzt erfolgen.

Des Weiteren kann die individualisierte, insbesondere mehrteilige, Negativform aus Metall bestehen, ausgewählt aus der Gruppe umfassend Leichtmetalle und Leichtmetalllegierung, insbesondere Aluminium, Magnesium, Buntmetalle und Buntmetalllegierung, insbesondere Messing und Bronze. Dadurch können Eigenschaften wie eine definierte Steifigkeit und eine definierte Wärmeleitfähigkeit gewährleistet sein, insbesondere hinsichtlich eines Wärmeeintrags zur Polymerisierung eines Zahnersatzes in der Negativform. Besonders vorteilhaft gestaltet sich bei der Verwendung von Metall der Wärmeeintrag, wenn das Komposit thermisch ausgehärtet wird. Die Festigkeit und Steifigkeit der Form erlauben hohe Pressdrücke. Des Weiteren kann Selektives Laser Sintering oder eine spanabhebende (reduktive) Bearbeitung bei einer Verwendung von Metall durchgeführt werden.

Zur Herstellung der individualisierten, insbesondere mehrteiligen, Negativform können Polymere eingesetzt werden ausgewählt aus der Gruppe umfassend Polyolefine, Polyoxmethylene, Polyamide, Polyurethane, Polyester, insbesondere PET, PLA, Polycaprolacton, Polycarbonate, Polystyrole, Acrylate, insbesondere Acrylat-Copolymere und Acrylat-Mischungen. Selbstverständlich können auch Mischungen der vorgenannten Polymere eingesetzt werden.

Bevorzugte Polymere, welche insbesondere transparent sind für eine Lichthärtung der Kompositrestauration, sind bspw. Polycarbonat (PC), Polystyrol (PS), Polymethylmethacrylat (PMMA) und deren Copolymere wie bspw. Styrol-Acrylnitril (SAN). Auf diese Weise kann die individualisierte, insbesondere mehrteilige, Negativform anforderungsspezifisch hergestellt und ausgestaltet werden.

Besonders bevorzugt können handelsübliche lichtpolymerisierbare Acryl-Harze eingesetzt werden, beispielsweise "Clear Guide" von EnvisionTec oder "SubG+" von Maker Juices Labs LLC.

Die vorgenannten Polymere können anorganische Komponenten und/oder Additive enthalten. Dies können feine Pulver (< 1 µm), Nanopartikel, Glaspulver, insbesondere Materialien mit eingestelltem Brechungsindex, Keramik, Glaskeramik, pyrogene Kieselsäure sein. Auf diese Weise kann die individualisierte, insbesondere mehrteilige, Negativform verstärkt werden, um de Pressdrücken besser stand zu halten, aber auch um vorteilhafte Transparenz für eine spätere Lichthärtung der Kompositrestauration zu gewährleisten

Die mittels des Verfahrens hergestellte individualisierte, insbesondere mehrteilige, Negativform kann lichtdurchlässig für Licht im sichtbaren Wellenlängenbereich und/oder im UV-Bereich und/oder im IR-Bereich sein. Unter "lichtdurchlässig" wird hier und im Folgenden ebenfalls partiell-lichtdurchlässig verstanden. "Lichtdurchlässig" ist nicht als zwingend vollständige Lichtdurchlässigkeit auszulegen. Auf diese Weis kann ein in die Negativform eingebrachtes Material, welches mittels Lichtinduzierter Polymerisation aushärtet, mittels Bestrahlung im entsprechenden Wellenlängenbereich ausgehärtet werden.

Wird die individualisierte Negativform einteilig ausgeführt, so kann sie Sollbruchstellen aufweisen, was ein Herausnehmen eines polymerisierten Materials, insbesondere eines individualisierten Zahnersatzes, aus der Negativform durch Aufbrechen an den Sollbruchstellen deutlich vereinfacht.

Des Weiteren betrifft die Erfindung ein Verfahren zur Herstellung eines Zahnersatzes mittels einer individualisierten, insbesondere mehrteiligen, Negativform, welche mit einem der vorgängig beschriebenen Verfahren hergestellt ist. Dabei wird Komposit-Material insbesondere schichtweise in die Negativform eingebracht. Dies kann mittels bekannter Ein- bzw. Auftragsverfahren, insbesondere mittels Extrusion eines Komposit-Materials, erfolgen. Auf diese Weise kann ein individualisierter Zahnersatz kosten- und zeiteffizient bereitgestellte werden.

Bei einem schichtweise Einbringen von Komposit-Material können sich die Komposit-Materialen für die Schichten in mindestens einer Eigenschaft ausgewählt aus der Gruppe umfassend Farbe, Transluzenz, Zusammensetzung von Füllmaterialien, mechanische Eigenschaften und Abrasionswiderstand unterscheiden. Auf diese Weise können beispielsweise verschiedenfarbige Komposit-Materialien eingearbeitet und so die Restauration dem natürlichen Zahn nachempfunden werden. Auf weitere Eigenschaften, wie insbesondere die vorgenannten, kann ebenfalls über die Zusammensetzung des Komposit-Materials Einfluss genommen werden.

Bei der Herstellung des Zahnersatzes kann das Komposit-Material im fliessfähigen Zustand in die individualisierte, insbesondere mehrteilige, Negativform unter Druck eingebracht werden. Auf diese Weise wird das Auftreten von Lufteinschlüssen vermieden. Dabei kann der Eintrag manuell oder maschinell erfolgen. Je nach Formmaterial kann der Druck bzw. Druckbereich variieren. Bei Formen aus Kunststoff bewegt sich der Druck im Bereich von 2 bis 20 bar, bevorzugt 4 bis 10 bar. Bei Formen aus Metall bewegt sich der Druck im Bereich mehrerer 100 bar. Hohe Pressdrücke erleichtern eine vollständige Formfüllung sowie Kompression/Austreiben von Gasblasen.

Bei der Herstellung des Zahnersatzes kann das in die individualisierte, insbesondere mehrteilige Negativform eingebrachte Komposit-Material mittels
- Temperatureintrag,
- Bestrahlung im sichtbaren Wellenlängenbereich, bevorzugt mit einer Wellenlänge von 430 nm bis 490 nm,
- Bestrahlung im UV-Wellenlängenbereich, bevorzugt mit einer Wellenlänge von 250nm bis 410 nm, oder
- Bestrahlung im IR-Wellenlängenbereich,
ausgehärtet werden. Die individualisierte, insbesondere mehrteilige, Negativform ist selbstverständlich entsprechend durchlässig für Licht in den entsprechenden Wellenlängenbereichen. Auf diese Weise wird das eingebrachte Komposit-Material ausgehärtet.

Bei der Herstellung von Zahnersatz können Verstärkungsfasern, insbesondere in Form einer Matte, in die individualisierte, insbesondere mehrteilige, Negativform vor dem Einbringen des Komposit-Materials vorgelegt werden. Selbstverständlich können als Verstärkungsfasern auch insbesondere röntgenopake Glasfasern eingesetzt werden. Des Weiteren können die Glasfasern mit einem Silan, bevorzugt Acryl-Silan oder Vinyl-Silan, modifiziert sein. Das Komposit-Material für die Herstellung des Zahnersatzes kann ein oder mehrere Additive, insbesondere zum Zwecke der Verstärkung, aufweisen. Mittels der vorgenannten Verstärkungs- und Modifikationselemente kann der Zahnersatz in seinen mechanischen Eigenschaften verstärkt werden.

Die Erfindung betrifft des Weiteren eine individualisierte, insbesondere mehrteilige, Negativform. Diese ist dabei insbesondere mit einem vorgängig beschriebenen Verfahren herstellbar und für ein Verfahren zur Herstellung eines Zahnersatzes wie vorgängig beschrieben geeignet. Dabei ist die individualisierte, insbesondere mehrteilige, Negativform eine Hohlform, insbesondere eine Spritzgussform.

Unter "individualisierte Negativform" versteht sich vorgängig, hier und im Folgenden eine Negativform, welche auf einer konkreten Zahn- und/oder Defektsituation eines Patienten basiert.

Unter "individualisiertem Zahnersatz" versteht sich vorgängig, hier und im Folgenden ein Zahnersatz, welcher auf einer konkreten Zahn- und/oder Defektsituation eines Patienten basiert.

Die individualisierte, insbesondere mehrteilige, Negativform kann eine oder mehrere Sollbruchstellen aufweisen. Auf diese Weise kann ein eingebrachtes und ausgehärtetes Komposit-Material, insbesondere bei einer einteiligen Form, vereinfacht entnommen werden.

Die Erfindung betrifft weiter einen individualisierten Zahnersatz hergestellt mittels einer individualisierten, insbesondere mehrteiligen Negativform, welche nach einem der vorbeschriebenen Verfahren herstellbar ist, und/oder mittels eines vorbeschriebenen Verfahrens zur Herstellung eine Zahnersatzes, und/oder mittels einer individualisierten, insbesondere mehrteiligen, Negativform aus Komposit-Material wie vorgängig beschrieben. Auf diese Weise kann ein individualisierter Zahnersatz kosten- und zeiteffizient bereitgestellt werden.

Des Weiteren kann der individualisierte Zahnersatz, welcher nach dem vorbeschriebenen Verfahren herstellbar ist eine Biegefestigkeit von ≥ 80 MPa gemäss ISO 4049 und/oder einem Biegemodul von mindestens 6000 MPA aufweisen. So weist der individualisierte Zahnersatz vorteilhafte mechanische Beständigkeit und Abrasionsbeständigkeit auf.

Ein weiterer Aspekt der Erfindung betrifft ein Behandlungsverfahren zur Zahnrestauration. Dabei umfasst das Behandlungsverfahren die Schritte
i) Präparation des defekten Zahnes, insbesondere Entfernen des zerstörten und/oder des mit Karies befallenden Gewebes,
ii) Erfassen digitaler Scandaten des Zahns in der Applikationsstelle und/oder der Defektstelle sowie dessen Eingliederung in den angrenzenden Teil des Zahnborgens oder den kompletten Zahnbogen mittels eines Interoralscanners,
iii) Konstruktion einer individualisierten, insbesondere mehrteiligen, Negativform basierend auf den erfassten Scandaten,
iv) Herstellung der individualisierten, insbesondere mehrteiligen, Negativform, insbesondere mit einem CNC-Verfahren, bevorzugt mit einem additiven Fertigungsverfahren,
v) Herstellung eines individualisierten Zahnersatzes mittels der individualisierten, insbesondere mehrteiligen Negativform,
vi) Einbringen des individualisierten Zahnersatzes in die Applikations- oder Defektstelle.

Auf diese Weise kann ein Behandlungsverfahren zur Verfügung gestellt werden, welches idealerweise innerhalb einer Patientensitzung durchgeführt werden kann, und dabei die vorteilhaften Eigenschaften der individualisierten, insbesondere mehrteiligen, Negativform, des individualisierten Zahnersatzes sowie deren Herstellungsverfahren kombinieren.

Bevorzugt kann der individualisierte Zahnersatz in die Applikations- oder Defektstelle mit einem Resinzement wie aus dem Bereich der Adhäsiv-Technik bekannt befestigt werden.

Zwischen dem Erfassen der Scandaten eines Zahns oder einer Defektstelle insbesondere mittels eines Interoralscanners (ii) und der Herstellung der individualisierten, insbesondere mehrteiligen, Negativform mit einem CNC-Verfahren basierend auf den erfassten Scandaten (iii) kann ein virtuelles 3-dimensionales Modell des individualisierten Zahnersatzes, und/oder der, insbesondere mehrteiligen, Negativform der Zahnsituation generiert werden. Dies ermöglicht neben der visuellen Darstellung eines derartigen Modells die Verwendung exakter Parameter/Dimensionen des individualisierten Zahnersatzes und/oder der, insbesondere mehrteiligen, Negativform. Die Konstruktion der vorgenannten 3-dimenionaler Modelle erfolgt mit Hilfe eines softwarebasierten Expertensystems auf Basis der erfassten Scandaten.

Die Erfindung wird im Folgenden anhand von Abbildungen exemplarischer Ausführungsbeispiele näher erläutert.
- Figur 1: Eine zweiteilige, erfindungsgemässe Negativform in einem offenen Zustand;
- Figur 2: eine Schnittansicht durch eine erfindungsgemässe Negativform in einem geschlossenen Zustand;
- Figur 3: eine Schrägansicht einer Formfläche der Negativform der Fig. 2;
- Figur 4: ein Fliessbild einer ersten Ausführungsform des erfindungsgemässen Verfahrens zur Herstellung einer individualisierten, insbesondere mehrteiligen, Negativform;
- Figur 5: ein Fliessbild einer zweiten Ausführungsform des erfindungsgemässen Verfahrens zur Herstellung einer individualisierten, insbesondere mehrteiligen, Negativform.

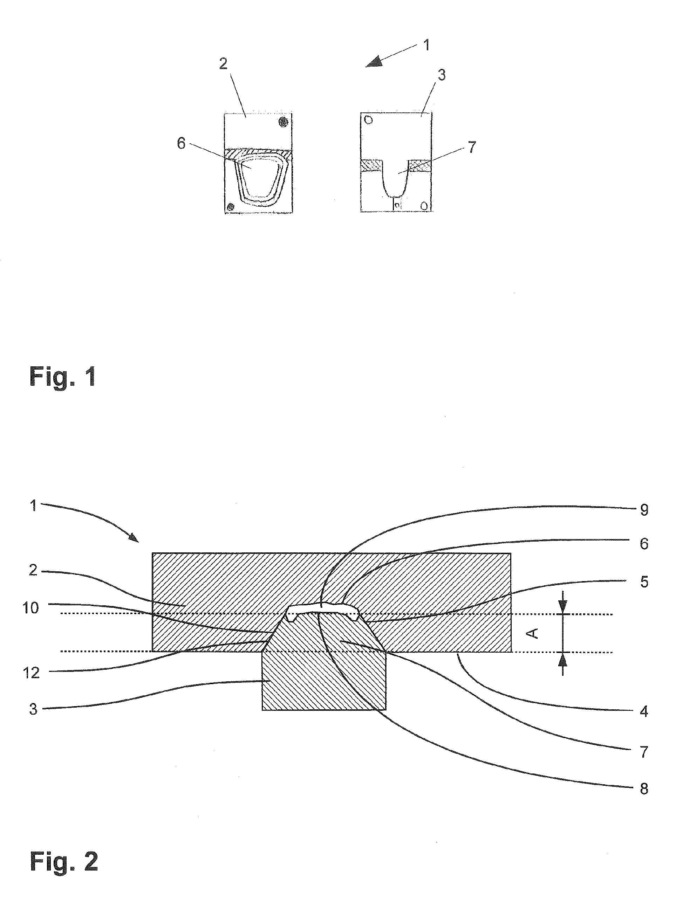

Figur 1 zeigt eine erfindungsgemässe Negativform 1 umfassend ein erstes Formenteil 2 und ein zweites Formenteil 3. Das Formenteil 2 weisst u.a. die Formfläche 6 auf. Das Formenteil 3 weisst u.a das Stempelelement 7 auf.

Figur 2 zeigt eine Schnittansicht durch eine erfindungsgemässe Negativform 1. Der Querschnitt liegt dabei senkrecht zu einer Längsrichtung des mit der Negativform 1 formbaren Zahnverblendungselements, wobei die Längsrichtung des Zahnverblendungselements als diejenige Richtung definiert ist, welche bei vorgesehener Applikation an einem Zahn parallel zu dessen axialer Richtung angeordnet ist.

Die Negativform 1 umfasst ein erstes Formenteil 2 und ein zweites Formenteil 3. An einer Trennfläche 4 des ersten Formenteils 2 ist eine Vertiefung 5 ausgebildet, in welcher eine erste Formfläche 6 des ersten Formenteils 2 angeordnet ist. Die Formfläche 6 dient dabei zur Ausbildung einer äusseren Oberfläche des Zahnverblendungselements, welche nach dessen Applikation im Wesentlichen sichtbar bleibt.

Das zweite Formenteil 3 umfasst ein Stempelelement 7, welches im geschlossenen Zustand der Negativform1 in der Vertiefung 5 angeordnet ist. Stirnseitig am Stempelelement 7 ist eine Formfläche 8 des zweiten Formenteils 3 ausgebildet. Die Formfläche 8 dient im Wesentlichen zur Ausbildung einer Innenseite des Zahnverblendungselements, mit welcher dieses im Wesentlichen auf einem Zahn bzw. einem Zahnstumpf appliziert wird.

Die Formflächen 6 und 8 begrenzen den Hohlraum 9 der Negativform1 zur Formung eines Zahnverblendungselements. Vorliegend ist das Stempelelement 7 einstückig mit dem Formenteil 3 ausgebildet.

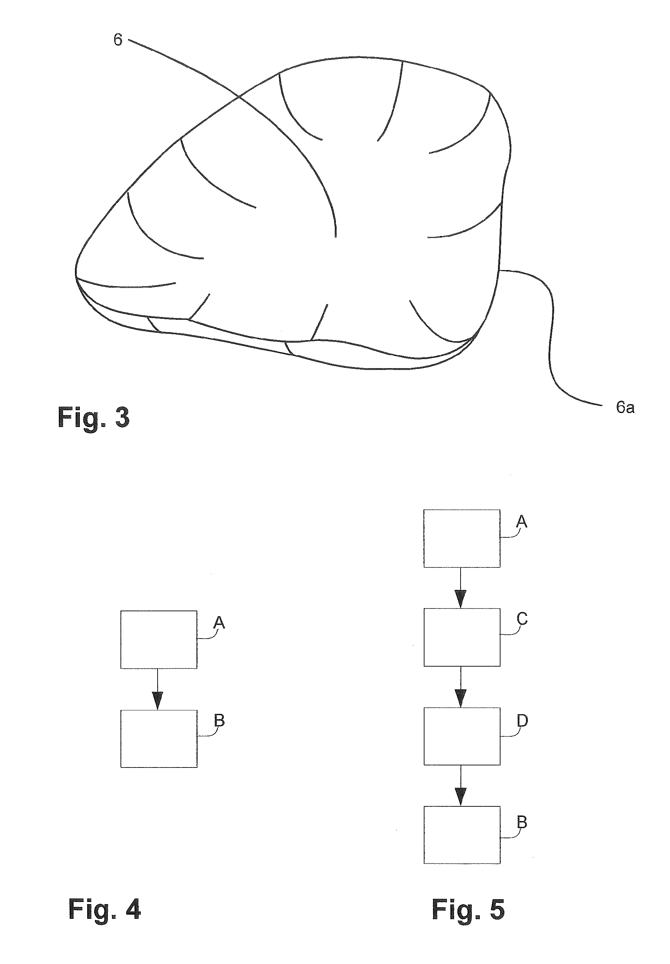

Ein Rand 6a (siehe bspw. Fig. 3) der Formfläche 6 des ersten Formenteils 2 ist in einem Abstand A von der Trennfläche 4, in das Formenteil 2 hinein versetzt, angeordnet. Ein Rand der Formfläche 8 des Formenteils 3 ist dabei unmittelbar beim Rand 6a der Formenfläche 6 angeordnet, sodass der Hohlraum 9 weitgehend vollständig von den Formflächen 6 und 8 umschlossen ist.

Der Flächenabschnitt 10 bildet eine Innenwand der Vertiefung 5 und erstreckt sich von einem Rand der Öffnung der Vertiefung 5 an der Trennfläche 4 bis zum Rand 6a der Formfläche 6 (vgl. Fig. 3). Im Querschnitt läuft der Flächenabschnitt 10 zur Formfläche 6 hin zusammen. Vorliegend ist eine Mantelfläche 12 des Stempelelements 7 komplementär zum Flächenabschnitt 10 ausgebildet.

Figur 3 zeigt zur besseren Illustration schematisch eine Ansicht der Formfläche 6 mit Rand 6a ohne weitere Elemente des Formenteils 2.

Figuren 4 und 5 veranschaulichen das erfindungsgemässe Verfahren zur Herstellung einer individualisierten, insbesondere mehrteiligen, Negativform in zwei Ausführungsformen. Figur 4 illustriert eine erste Ausführungsform des Verfahrens. In einem ersten Schritt A werden digitale Scandaten eines Zahns oder einer Defektstellen insbesondere mittels eines Interoralscanners erfasst. Anschliessend wird in einem weiteren Schritt B eine individualisierte, insbesondere mehrteilige, Negativform mit einem CNC-Verfahren basierend auf den erfassten Scandaten des ersten Schritts A hergestellt. Figur 5 zeigt eine zweite Ausführungsform, wobei zwischen die Schritte Erfassen von Scandaten A und Herstellen der Negativform B weitere Schritte durchgeführt werden. Diese sind ein Nachbearbeiten C der erfassten Scandaten mit geeigneten Softwarelösungen (Software-basierte Expertensysteme), insbesondere auf automatisierte Art und Weise, und Generieren D eines virtuellen 3-dimensionalen Modells der Zahnsituation und/oder der Negativform der Zahnsituation.

## Patentansprüche

1. Verfahren zur Herstellung einer individuellen, insbesondere mehrteiligen, Negativform für die Herstellung von Zahnersatz umfassend die Schritte
i) Erfassen von digitalen Scandaten eines Zahns oder einer, insbesondere präparierten, Defektstelle insbesondere mittels eines Interoralscanners, und
ii) Herstellen der individualisierten, insbesondere mehrteiligen, Negativform mit einem computer-gesteuertem Verfahren, insbesondere einem CNC-Verfahren, basierend auf den erfassten Scandaten.

2. Verfahren gemäss Anspruch 1 **dadurch gekennzeichnet, dass** zwischen den Schritten i) und ii) der Schritt Konstruieren eines 3-dimensionalen Modells eines individualisierten Zahnersatzes und/oder eines 3-dimensionalen Modells der individualisierten, insbesondere mehrteiligen, Negativform, insbesondere mittels eines softwarebasierten Expertensystems, auf Basis der erfassen Scandaten durchgeführt wird.

3. Verfahren gemäss Anspruch 1 oder 2 **dadurch gekennzeichnet, dass** das computer-gesteuerte Verfahren, insbesondere das CNC-Verfahren, ein abtragendes Verfahren, insbesondere ein Fräs-, Schleif- oder Strahl-Verfahren, ist.

4. Verfahren gemäss Anspruch 1 oder 2, **dadurch gekennzeichnet, dass** das computer-gesteuerte Verfahren, insbesondere das CNC-Verfahren, ein additives Verfahren, insbesondere Stereolitographie, selektives Lasersintern, selektives Laserschmelzen, digital light processing, polyjet printing, multijet modeling, fused deposition molding, ist.

5. Verfahren gemäss einem der Ansprüche 1 bis 4 **dadurch gekennzeichnet, dass** die individualisierte, insbesondere mehrteilige, Negativform aus Metall besteht, ausgewählt aus der Gruppe umfassend Leichtmetalle und Leichtmetalllegierung, insbesondere Aluminium, Magnesium, Buntmetalle und Buntmetalllegierung, insbesondere Messing und Bronze.

6. Verfahren einem der Ansprüche 1 bis 4, **dadurch gekennzeichnet, dass** die individualisierte, insbesondere mehrteilige, Negativform Polymere umfasst, ausgewählt aus der Gruppe umfassend Polyolefine, Polyoxmethylene, Polyamide, Polyurethane, Polyester, insbesondere PET, PLA, Polycaprolacton, Polycarbonate, Polystyrole, Polymethylmethacrylate, insbesondere deren Copolymere wie Styrol-Acrylnitril, Acrylate, insbesondere Acrylat-Copolymere und Acrylat-Mischungen (blends).

7. Verfahren gemäss Anspruch 6 **dadurch gekennzeichnet, dass** die Polymere anorganische Komponenten und/oder Additive, wie feine Pulver (< 1 µm), Nanopartikel, Glaspulver, insbesondere Materialien mit eingestelltem Brechungsindex, Keramik, Glaskeramik, pyrogene Kieselsäure aufweisen.

8. Verfahren gemäss Anspruch 6 oder 7 **dadurch gekennzeichnet, dass** die individualisierte, insbesondere mehrteilige, Negativform lichtdurchlässig für Licht im sichtbaren Wellenlängenbereich und/oder im UV-Bereich und/oder im IR-Bereich ist.

9. Verfahren zur Herstellung eines Zahnersatzes mittels einer individualisierten, insbesondere mehrteiligen, Negativform hergestellt mit einem Verfahren insbesondere gemäss einem der Ansprüche 1, 2, 4, 6, 7 oder 8, **dadurch gekennzeichnet, dass** Komposit-Material insbesondere schichtweise in die Negativform eingebracht wird.

10. Verfahren gemäss Anspruch 9, **dadurch gekennzeichnet, dass** bei einem schichtweisen Einbringen von Komposit-Material sich die Komposit-Materialen für die Schichten in mindestens einer Eigenschaft ausgewählt aus der Gruppe umfassend Farbe, Transluzenz, Zusammensetzung von Füllmaterialien, mechanische Eigenschaften und Abrasionswiderstand unterscheiden.

11. Verfahren gemäss Anspruch 9 oder 10 **dadurch gekennzeichnet, dass** das Komposit-Material im fliessfähigen Zustand in die individualisierte, insbesondere mehrteilige Negativform unter Druck eingebracht wird.

12. Verfahren gemäss einem der Ansprüche 9 bis 11 **dadurch gekennzeichnet, dass** das in die individualisierte, insbesondere mehrteilige Negativform eingebrachte Komposit-Material mittels
- Temperatureintrag,
- Bestrahlung im sichtbaren Wellenlängenbereich, bevorzugt mit einer Wellenlänge von 430 nm bis 490 nm,
- Bestrahlung im UV-Wellenlängenbereich, bevorzugt mit einer Wellenlänge von 250nm bis 410 nm, oder
- Bestrahlung im IR-Wellenlängenbereich,
ausgehärtet wird.

13. Individualisierte, insbesondere mehrteilige, Negativform insbesondere herstellbar mit einem Verfahren gemäss Anspruch 1 bis 8 für die Verwendung bei der Herstellung eines Zahnersatzes, insbesondere gemäss einem der Ansprüche 9 bis 12, **dadurch gekennzeichnet, dass** die individualisierte, insbesondere mehrteilige, Negativform eine Hohlform, insbesondere eine Spritzgussform, ist.

14. Individualisierter Zahnersatz hergestellt mittels einer individualisierten, insbesondere mehrteiligen Negativform insbesondere herstellbar nach einem Verfahren gemäss einem der Ansprüche 1 bis 8, und/oder mittels eines Verfahrens, insbesondere gemäss einem der Ansprüche 9 bis 12, und/oder mittels einer individualisierten, insbesondere mehrteiligen, Negativform aus Komposit-Material insbesondere gemäss Anspruch 13.

15. Individualisierter Zahnersatz gemäss Anspruch 14 **dadurch gekennzeichnet, dass** der individualisierte Zahnersatz eine Biegefestigkeit von ≥ 80 MPa gemäss ISO 4049 und/oder ein Biegemodul von mindestens 6000 MPA aufweist.
